# EUROPÄISCHE PATENTSCHRIFT

(11) **EP 1 249 230 B1**
(45) Veröffentlichungstag und Bekanntmachung des Hinweises auf die Patenterteilung: **05.11.2003**
(21) Anmeldenummer: 01109131.1
(22) Anmeldetag: 12.04.2001
(51) Int. Cl.: A61K 9/107, A61K 31/122

(54) **Coenzym Q10 enthaltende Mikroemulsion-Prekonzentrate und Mikroemulsionen**
Microemulsion-preconcentrates and microemulsions comprising coenzyme Q10
Microémulsions et préconcentrés en microémulsion comprenant de la coenzyme Q10

(43) Veröffentlichungstag der Anmeldung: 16.10.2002
(73) Patentinhaber: VESIFACT AG, 6342 Baar 2 (CH)
(72) Erfinder: Supersaxo, Andreas, Werner, 6340 Baar (CH); Weder, Marc, Antoine, 8803 Rüschlikon (CH); Weder, Hans, Georg, 8803 Rüschlikon (CH)
(74) Vertreter: Hepp, Dieter

(56) Entgegenhaltungen:
- WO-A-01/28520
- DE-A- 4 405 545
- KOMMURU TR ET AL: "Self-emulsifying drug delivery systems (SEDDS) of coenzyme Q10: Formulation development and bioavailability assessment." INTERNATIONAL JOURNAL OF PHARMACEUTICS (KIDLINGTON), Bd. 212, Nr. 2, 16. Januar 2001 (2001-01-16), Seiten 233-246, XP001025175 ISSN: 0378-5173
- DATABASE WPI Section Ch, Week 198728 Derwent Publications Ltd., London, GB; Class A96, AN 1987-194640 XP002177273 & JP 62 123113 A (GREEN CROSS CORP), 4. Juni 1987 (1987-06-04)
- TAKEUCHI H ET AL: "Improvement of photostability of ubidecarenone in the formulation of a novel powdered dosage form termed redispersible dry emulsion." INTERNATIONAL JOURNAL OF PHARMACEUTICS (AMSTERDAM), Bd. 86, Nr. 1, 1992, Seiten 25-33, XP001025186 ISSN: 0378-5173
- KIBBE AH (ED.): "Handbook of Pharmaceutical Excipients, 3rd Edition", 2000, PHARMACEUTICAL PRESS, LONDON

## Beschreibung

Die vorliegende Erfindung bezieht sich auf neue Formulierungen in Form von Mikroemulsion-Prekonzentraten und Mikroemulsionen, sowie deren Verwendung als Trägersystem für in Wasser schwerlösliche Wirkstoffe aus der Klasse der Ubichinone, gegebenenfalls auch in Kombination mit Vitaminen und Spurenelementen. Die erfindungsgemässen Formulierungen eignen sich besonders zur oralen Verabreichung in Form von Einheitsdosisformen.

Ubichinone können in fast allen Organismen in grösseren Mengen nachgewiesen werden; die einzigen Ausnahmen sind gram-positive und Cyanobakterien. Ubichinone werden je nach Zahl der in der Seitenkette verknüpften Isopren-Einheiten als Q1, Q2, Q3 usw. bezeichnet. Sie treten bevorzugt mit bestimmten Kettenlängen auf, zum Beispiel in einigen Mikroorganismen und Hefen mit n=6. Bei den meisten Säugetieren, einschliesslich des Menschen, überwiegt das Coenzym Q10, auch als Ubidecarenon bezeichnet. Der menschliche Organismus synthetisiert einen Teil seines Coenzym Q10-Bedarfes selbst, der Rest wird über die Nahrung aufgenommen. Mit zunehmendem Alter nimmt die endogene Produktion von Coenzym Q10 kontinuierlich ab.

Die vielfältigen Wirkungen des Coenzym Q10 beruhen sowohl auf seinen biologischen Funktionen im Energiehaushalt der Zellen als auch auf seinen antioxidativen Eigenschaften. Aufgrund dieser Wirkungen wird Coenzym Q10 zur Prophylaxe und/oder Behandlung folgender Erkrankungen eingesetzt:
- Herz- und Kreislauferkrankungen wie Herzinfarkt, Angina pectoris, Atherosclerosis und Bluthochdruck,
- Degenerative Erkrankungen des Zentralnervensystems wie Alzheimer, Parkinson und Depressionen,
- Zahnfleischerkrankungen
- Muskuläre Dystrophie
- Männliche Infertilität,
- zur Stärkung des Immunsystems und
zur Verbesserung der körperlichen Leistungsfähigkeit. Des weiteren kann Coenzym Q10 Nebenwirkungen gewisser Arzneimittel verhindern oder reduzieren, so z.B. diejenigen von Statinen wie Lovastatin, Pravastatin und Simvastatin oder von Zytostatika wie Doxorubicin.

Coenzym Q10 ist eine lipophile (d.h. hydrophobe) Substanz mit sehr geringer Wasserlöslichkeit (praktisch Wasser unlöslich). Formulierungen von Coenzym Q10, z.B. für eine orale Verabreichung, beruhen daher vorwiegend auf der Anwendung von Ölen oder ähnlichen Hilfsstoffen als Trägermedien. Die so formulierten und gegenwärtig im Handel erhältlichen, oral zu applizierenden Präparate wie z.B. Super Bio-Quinone (Pharma Nord), Bio Coenzyme Q10 (Solanova) und Q-Gel Ultra (Tishcon) weisen eine sehr geringe Bioverfügbarkeit auf.

Kommuru et al. (Int. J. Pharm. 212 (2001): 233-246) beschreiben selbstemulgierbare Systeme zur Verabreichung von Coenzym Q10.

Aufgabe der vorliegenden Erfindung ist es, eine Formulierung zu entwickeln, welche die Bioverfügbarkeit von Coenzym Q10 verbessert. Ueberraschend wurde gefunden, dass bei einer Verabreichung von Formulierungen auf der Basis eines Mikroemulsion-Prekonzentrates die orale Bioverfügbarkeit von Coenzym Q10 im Vergleich zu den oben erwähnten kommerziell erhältlichen Präparaten signifikant höher ist.

Unter dem erfindungsgemässen Mikroemulsion-Prekonzentrat wird ein System verstanden, das beim Kontakt mit Wasser oder einem anderen wässrigen Medium wie künstlicher Magen-oder Darmsaft, z.B. bei Zugabe zu Wasser, eine Mikroemulsion ergibt. Bei einer solchen Mikroemulsion handelt es sich im herkömmlich anerkannten Sinn um eine nicht-opaque oder praktisch nicht-opaque kolloidale Dispersion, welche Wasser und organische Komponenten unter Einschluss lipophiler (d.h. hydrophober) Komponenten enthält.

Mikroemulsionen im Sinne der Erfindung lassen sich dadurch erkennen, dass sie eine oder mehrere der folgenden Eigenschaften aufweisen:
- Sie werden spontan gebildet, wenn ihre Komponenten miteinander in Kontakt gebracht werden; es ist also hierzu praktisch keine Zufuhr von Energie notwendig, und die Bildung solcher Mikroemulsionen erfolgt daher ohne Erhitzen oder Anwendung einer hohen Scherkraft oder einer anderen wesentlichen Durchmischung.
- Sie sind praktisch nicht opaque, nämlich transparent oder opaleszent, wenn sie unter einem optischen Mikroskop betrachtet werden. Sie sind in ihrem nichtgestörten Zustand optisch isotrop, obwohl sich bei einer Beobachtung beispielsweise unter Anwendung einer Röntgenstrahltechnik eine anisotrope Struktur feststellen lässt.
- Sie enthalten eine disperse oder stückige (Tröpfchen-) Phase, deren Teilchen eine Grösse von weniger als 200 nm haben, wovon ihre optische Transparenz herrührt. Die Teilchen können kugelig sein oder auch sonstige Strukturen haben; beispielsweise können sie flüssige Kristalle mit lamellaren, hexagonalen oder isotropen Symmetrien sein. Im allgemeinen enthalten Mikroemulsionen Tröpfchen oder Teilchen mit einer maximalen Abmessung, beispielsweise einem Durchmesser, von weniger als 150 nm, gewöhnlich etwa 10-100 nm.

Bei den erfindungsgemässen Mikroemulsion-Prekonzentraten handelt es sich demgemäss um galenische Systeme, die einen in Wasser schwerlöslichen therapeutischen Wirkstoff aus der Klasse der Ubiquinone enthalten und beim Zusammenbringen mit Wasser bzw.

Magen- und Darmsaft spontan oder praktisch spontan, d.h. ohne nennenswerten Energieeintrag, zur Bildung einer Mikroemulsion befähigt sind.

Gegenstand der Erfindung ist eine Zusammensetzung in Form eines Mikroemulsion-Prekonzentrates enthaltend
(a) eine Mischung bestehend aus einem Triglycerid und einer Omega-9-Fettsäure und/oder einer Omega-6-Fettsäure; und
(b) eine oberflächenaktive Komponente enthaltend ein Tensid, insbesondere vom Polyoxyethylen-Typ,
(c) ein Wirkstoffgemisch enthaltend ein Ubiquinon, vorzugsweise Q10, in Kombination mit Vitaminen, vorzugsweise Vitamin E und dessen Derivate, und/oder Spurenelementen, wobei das Ubiquinon in (a) und/oder (b) lösbar ist.

Gegenstand der Erfindung sind auch Brausetablette und Granulate, enthaltend eine Zusammensetzung in Form eines Mikroemulsion-Prekonzentrates enthaltend
(a) eine Mischung bestehend aus einem Triglycerid und einer Omega-9-Fettsäure und/oder einer Omega-6-Fettsäure; und
(b) eine oberflächenaktive Komponente enthaltend ein Tensid, insbesondere vom Polyoxyethylen-Typ,
(c) einen Wirkstoff ausgewählt aus der Klasse der Ubiquinone, wobei der Wirkstoff in (a) und/oder (b) lösbar ist.

Die erfindungsgemässen Mikroemulsion-Prekonzentrate sind vorzugsweise dadurch gekennzeichnet, dass sie
(a) eine Mischung bestehend aus einem mittelkettigen Triglycerid und einer Omega-9-Fettsäure und/oder einer Omega-6-Fettsäure,
(b) eine oberflächenaktive Komponente enthaltend ein Tensid vom Polyoxyethylen-Typ umfassen und
(c) einen in Wasser schwerlöslichen, in Komponente (a) und/oder (b) jedoch löslichen, therapeutischen Wirkstoff aus der Klasse der Ubiquinone
enthalten.

Das Verhältnis der Bestandteile (a) : (b) : (c), (a) : (c) beziehungsweise (b) : (c) der erfindungsgemässen Mikroemulsion muss selbstverständlich so gewählt werden, dass der Wirkstoff (c) stabil solubilisiert ist, d.h. es dürfen über mehrere Wochen keine Präzipitate auftreten.

Im Gegensatz zu den Formulierungen des Stands der Technik sind die Mikroemulsion-Prekonzentrate der vorliegenden Erfindung im wesentlichen frei von mit Wasser mischbaren oder in Wasser löslichen Komponenten. Dabei handelt es sich insbesondere um die Komponenten
- C₁-C₅-Alkyl- oder Tetrahydrofurfuryl-Diether oder -Teilether niedermolekularer Mono- oder Polyoxy-C₂-C₁₂-Alkandiole;
- 1,2-Propylenglykol;
- niedere Alkanole;
- Veresterungsprodukte von Polycarbonsäuren mit 2-10, insbesondere 3-5 Carboxylgruppen mit C₁-C₁₀-Alkoholen; und
- Veresterungsprodukte von Polyolen mit 2-10, insbesondere 3-5 Carboxylgruppen mit C₂-C₁₁-Carbonsäuren;
insbesondere im wesentlichen frei von Diethylenglykolmonomethylether, Glycofurol, 1,2-Propylenglykol, Triethylcitrat, Tributycitrat, Acetyltributycitrat, Acetyltriethylcitrat, Triacetin, Ethanol, Polyethylenglykol, Dimethylisosorbit und Propylencarbonat.

Im Gegensatz zu den einschlägigen Formulierungen gemäss WO 98/40051 A enthält Komponente (a) der erfindungsgemässen Mikroemulsion-Prekonzentrat zusätzlich zu einem mittelkettigen Triglycerid eine Omega-9-Fettsäure und/oder eine Omega-6-Fettsäure, womit überraschenderweise eine besonders ausgeprägte Stabilität der erfindungsgemässen Mikroemulsionen verbunden ist, welche für deren therapeutische Verwertbarkeit von ausschlaggebender Bedeutung ist.

Die erfindungsgemässen Mikroemulsion-Prekonzentrate können hergestellt werden, indem man die einzelnen Bestandteile, gegebenenfalls unter Erwärmen, innig miteinander vermischt. Die Mikroemulsion-Prekonzentrate können auch hergestellt werden, indem man die Komponente (b) unter Rühren, gegebenenfalls unter Erwärmen, in der Komponente (a) löst, und die erhaltene Lösung unter weiterem Rühren mit der Komponente (c) versetzt. Hierbei ist es von besonderer Bedeutung, dass die Komponente beziehungsweise der Wirkstoff (c) entweder in Komponente (a) oder Komponente (b) oder aber in beiden Komponenten (a) und (b) lösbar ist und dass beim Herstellen des Pre-Konzentrates, d.h. dem Gemisch aus allen drei Komponenten (a), (b) und (c) der Wirkstoff in jedem Fall weiterhin in gelöster Form vorliegt.

Als Komponente (a) eignen sich Mischungen aus einem mittelkettigen Fettsäuretriglycerid, zweckmässigerweise einem Fettsäuretriglycerid, in welchem die Fettsäurereste 4 bis 18, vorzugsweise 6 bis 18 C-Atome aufweisen, und einer Omega-9- und/oder einer Omega-6-Fettsäure. Diese Substanzen sind mit Wasser nicht mischbar oder in Wasser unlöslich bzw. praktisch unlöslich und weisen keine oder praktisch keine oberflächenaktive Funktion auf.

Bevorzugte mittelkettige Fettsäuretriglyceride sind Capryl-/Caprinsäure-Triglyceride, wie sie beispielsweise unter der Warenbezeichnung MIGLYOL bekannt und im Handel erhältlich sind (Fiedler, Lexikon der Hilfsstoffe, 3. Auflage, Seiten 808 bis 809, 1989). Hierzu gehören beispielsweise die folgenden Produkte:

### MIGLYOL 810, 812 und 818

Hierbei handelt es sich um ein fraktioniertes Kokosnussöl, das Triglyceride von Capryl- und Caprinsäure enthält und ein Molekulargewicht von etwa 520 (MIGLYOL 810 und 812) beziehungsweise 510 (MIGLYOL 818) hat. Es weist eine Fettsäure-Zusammensetzung mit C₆ von maximal 2 Prozent (MIGLYOL 810) und 3 Prozent (MIGLYOL 812 und 818) auf, mit C₈ etwa von 65 bis 75 Prozent (MIGLYOL 810), 50 bis 65 Prozent (MIGLYOL 812) und 45 bis 60 Prozent (MIGLYOL 818). C₁₀ ist bei MIGLYOL mit 25 bis 35 Prozent, bei MIGLYOL 812 mit etwa 30 bis 45 Prozent und bei MIGLYOL 818 etwa 25 bis 40 Prozent vertreten, C₁₂ mit maximal 2 Prozent (MIGLYOL 810), 5 Prozent (MIGLYOL 812) und 2 bis 5 Prozent (MIGLYOL 818). MIGLYOL 818 weist zusätzlich noch einen Anteil an C_{18:2} von etwa 4 bis 6 Prozent auf.

Ferner sind auch Triglyceride von Caprylsäure und Caprinsäure geeignet, wie sie unter der Warenbezeichnung MYRITOL bekannt und erhältlich sind (Fiedler, Lexikon der Hilfsstoffe, 3. Auflage, Seite 834, 1989). Hierzu gehört beispielsweise das Produkt MYRITOL 813.

Weitere geeignete Produkte dieser Klasse sind CAPTEX 355, CAPTEX 300, CAPTEX 800, CAPMUL MCT, NEOBEE M5 und MAZOL 1400.

Geeignete Omega-9-Fettsäuren sind hauptsächlich solche mit 12-24, insbesondere 16-24, vorzugsweise 18-22 C-Atomen, beispielsweise Oelsäure und Eicosatriensäure. Besonders bevorzugt ist die Oelsäure.

Geeignete Omega-6-Fettsäuren sind hauptsächlich solche mit 12-24, insbesondere 16-24, vorzugsweise 18-22 C-Atomen, beispielsweise Linolsäure, gamma-Linolensäure, dihommo-gamma-Linolensäure und Arachidonsäure. Besonders bevorzugt ist die Linolsäure.

In einer besonders bevorzugten Ausführungsform verwendet man als Komponente (a) eine Mischung bestehend aus einem Capryl-/Caprinsäure-Triglycerid, Oelsäure und/oder Linolsäure.

Komponente (c), der in Wasser schwerlösliche, in Komponente (a) und/oder (b) jedoch lösliche therapeutische Wirkstoff aus der Klasse der Ubiquinone, ist vorzugsweise Coenzym Q10; es kann aber auch ein anderes geeignetes Ubiquinon, gegebenenfalls in Kombination mit Vitaminen, vorzugsweise Vitamin E, und/oder Spurenelementen verwendet werden.

Bei Komponente (b), der oberflächenaktiven Komponente enthaltend ein Tensid vom Polyoxyethylen-Typ, kann es sich um ein hydrophiles oberflächenaktives Mittel oder um ein lipophiles oberflächenaktives Mittel handeln, doch kommen auch Gemische solcher Mittel in Frage.

Beispiele für solche Tenside sind folgende:
- Reaktionsprodukte von natürlichen oder hydrierten Pflanzenölen und Ethylenglykol, nämlich polyoxyethylenglykolierte natürliche oder hydrierte Pflanzenöle, wie polyoxyethylenglykolierte natürliche oder hydrierte Rizinusöle. Besonders geeignet sind die verschiedenen Tenside, die unter der Bezeichnung CREMOPHOR bekannt und erhältlich sind (Fiedler, Lexikon der Hilfsstoffe, 3. Auflage, Seiten 326 bis 327, 1989), vor allem die Produkte mit den Bezeichnungen CREMOPHOR RH 40, CREMOPHOR RH 60 und CREMOPHOR EL. Ferner eignen sich als solche Produkte auch die verschiedenen Tenside, die unter der Bezeichnung NIKKOL bekannt und erhältlich sind, beispielsweise NIKKOL HCO-60.
- Polyoxyethylensorbitanfettsäureester, beispielsweise die Mono-und Trilaurylester, die Mono- und Tripalmitylester, die Mono- und Tristearylester und die Mono- und Trioleylester, wie sie unter der Bezeichnung TWEEN bekannt und erhältlich sind (Fiedler, Lexikon der Hilfsstoffe, 3. Auflage, Seiten 1300 bis 1304, 1989), beispielsweise die Produkte
   TWEEN 20: Polyoxyethylen(20)sorbitanmonolaurat,
   TWEEN 40: Polyoxyethylen(20)sorbitanmonopalmitat,
   TWEEN 60: Polyoxyethylen(20)sorbitanmonostearat,
   TWEEN 80: Polyoxyethylen(20)sorbitanmonooleat,
   TWEEN 65: Polyoxyethylen(20)sorbitantristearat,
   TWEEN 85: Polyoxyethylen(20)sorbitantrioleat,
   TWEEN 21: Polyoxyethylen(4)sorbitanmonolaurat,
   TWEEN 61: Polyoxyethylen(4)sorbitanmonostearat und
   TWEEN 81: Polyoxyethylen(4)sorbitanmonooleat.

Besonders bevorzugt aus dieser Klasse von Verbindungen ist TWEEN 80.
- Polyoxyethylenfettsäureester, beispielsweise die im Handel unter der Bezeichnung MYRJ bekannten und erhältlichen Polyoxyethylenstearinsäureester (Fiedler, Lexikon der Hilfsstoffe, 3. Auflage, Seite 834, 1989), insbesondere das Produkt MYRJ 52, sowie auch die unter der Bezeichnung CETIOL HE bekannten und erhältlichen Polyoxyethylenfettsäureester (Fiedler, Lexikon der Hilfsstoffe, 3. Auflage, Seite 284, 1989).
- Copolymerisate von Polyoxyethylen und Polyoxypropylen, wie sie beispielsweise unter den Bezeichnungen PLURONIC und EM-KALYX bekannt und erhältlich sind (Fiedler, Lexikon der Hilfsstoffe, 3. Auflage, Seiten 956 bis 958, 1989), insbesondere das Produkt PLURONIC F68.
- Blockcopolymerisate von Polyoxyethylen und Polyoxypropylen, wie sie beispielsweise unter der Bezeichnung POLOXAMER bekannt und erhältlich sind (Fiedler, Lexikon der Hilfsstoffe, 3. Auflage, Seite 959, 1989), insbesondere das Produkt POLOXAMER 188.
- Polyethoxylierte Vitamin E Derivate, insbesondere das Produkt VITAMIN E TPGS (d-Alpha Tocoperyl Polyethylene Glycol 1000 Succinate, Eastman).
- Polyethoxylierte Hydroxyfettsäureester, insbesondere das Produkt SOLUTOL HS 15 (Polyoxyethylen-660-Hydroxystearat, BASF).
- Umesterungsprodukte von natürlichen Pflanzenölglyceriden und Polyethylenpolyolen. Hierzu gehören Umesterungsprodukte aus verschiedenen, beispielsweise nichthydrierten, Pflanzenölen, wie Maisöl, Kernöl, Mandelöl, Erdnussöl, Olivenöl und Palmenöl, sowie aus Gemischen hiervon mit Polyethylenglykolen, insbesondere mit solchen, die ein mittleres Molekulargewicht von 200-800 haben. Verschiedene derartige Umesterungsprodukte sind unter der Bezeichnung LABRAFIL bekannt und erhältlich (Fiedler, Lexikon der Hilfsstoffe, 3. Auflage, Seite 707, 1989); hiervon sind die Produkte LABRAFIL M 1944 CS und LABRAFIL M 2130 CS besonders geeignet.

Ethylenoxidaddukte von Sterolen und Derivaten davon, beispielweise von Cholesterol und Derivaten hiervon, wie Produkte, die von Sitosterol, Campesterol, oder Stigmasterol abgeleitet sind, beispielsweise von Sojasterolen und Derivaten hiervon (Fiedler, Lexikon der Hilfsstoffe, 3. Auflage, Seiten 554 und 555, 1989), wie sie unter den Bezeichnungen GENEROL bekannt und erhältlich sind, insbesondere die Produkte GENEROL 122 E5, 122 E10 und 122 E25.

Die erfindungsgemässen Mikroemulsion-Prekonzentrate umfassen sowohl Systeme, die ein einzelnes oberflächenaktives Mittel enthalten, als auch Systeme, die ein Gemisch von zwei oder mehreren oberflächenaktiven Mitteln enthalten, z.B. TWEEN 80 + CREMOPHOR RH 40, TWEEN 80 + CREMOPHOR RH 40 + VITAMIN E TPGS etc.

Erfindungsgemäss verwendet man vorzugsweise eine oberflächenaktive Komponente, welche einen Polyoxyethylensorbitanfettsäureester, ein polyoxyethylenglykoliertes natürliches oder hydriertes Pflanzenöl oder Mischungen davon enthält.

Die erfindungsgemässen Mikroemulsion-Prekonzentrate können auch noch weitere Stoffe enthalten, wie z.B. Antioxidantien, Verdikkungsmittel, Riech- und/oder Geschmacksstoffe, Farbstoffe, etc.

Die erfindungsgemässen Pre-Mikroemulsionen sind in erster Linie für eine orale Anwendung gedacht. Bevorzugt wird dabei die sogenannte Einheitsdosisform, d.h. das Mikroemulsion-Prekonzentrat wird in einen Formkörper wie eine Weich- oder Hartkapsel z.B. aus Gelatine oder Stärke, verbracht. Wenn die den Wirkstoff enthaltende Pre-Mikroemulsion freigesetzt wird, bildet sich spontan in Verbindung mit Magen-Darmflüssigkeit eine Mikroemulsion. Die erfindungsgemässen Zusammensetzungen erweisen sich für eine orale Verabreichung in Form von Einheitsdosisformem auch deshalb als besonders geeignet, weil eine Zugabe von flüchtigen organischen Lösungsmitteln, insbesondere vom häufig verwendeten Ethanol nicht erforderlich ist. Beim Einsatz der genannten Lösungsmitteln wird durch deren Verdampfen durch die Aussenwand des Formkörpers, insbesondere der Weich- oder Hartgelatinekapsel, die Lagerfähigkeit negativ beeinträchtigt und der Wirkstoff kristallisiert aus. Das Eintreten dieser negativen Effekte muss durch aufwendige Massnahmen bei der Verpackung und Lagerung vermieden werden.

Die neuen Zusammensetzungen lassen sich auch zu Brausetabletten oder als Granulaten weiterverarbeiten.

Eine Einheitsdosisform der oben beschriebenen Art enthält zweckmässigerweise 0,5 bis 25, vorzugsweise 10-20 Gewichtsprozent eines in Wasser schwerlöslichen, in Komponente (a) und/oder (b) jedoch löslichen therapeutischen Wirkstoffs der Klasse der Ubichinone (Komponente (c)), 9,5 bis 70, vorzugsweise 20 bis 70 Gewichtsprozent und weiter bevorzugt 25 bis 65 Gewichtsprozent einer Mischung bestehend aus einem mittelkettigen Triglycerid und einer Omega-9-Fettsäure und/oder einer Omega-6-Fettsäure (Komponente (a)) und 20 bis 90, vorzugsweise 25 bis 65 Gewichtsprozent der oberflächenaktiven Komponente (b).

Durch die vorliegende Erfindung lassen sich auch pharmazeutische Zusammensetzungen bereitstellen, die einen in Wasser schwerlöslichen, in Komponente (a) jedoch löslichen therapeutischen Wirkstoff aus der Klasse der Ubiquinone enthalten und die selbst Mikroemulsionen darstellen; in diesen Mikroemulsionen ist der Wirkstoff stabil solubilisiert, wobei über mehrere Wochen keine Präzipitate beobachtet werden. Für eine orale Verabreichung können Mikroemulsionen, die man beispielsweise durch Verdünnen der erfindungsgemässen Mikroemulsion-Prekonzentrate mit Wasser oder einem wässrigen Medium erhält, direkt als Trinkformulierungen verwendet werden. Ist eine parenterale Anwendung vorgesehen, dann enthalten Zusammensetzungen, in denen weitere Hilfsstoffe vorhanden sein können, ebenfalls Wasser, so dass sich eine wässrige Mikroemulsion in Form einer Injektionslösung, einer Infusionslösung oder dergleichen ergibt.

Solche pharmazeutischen Zusammensetzungen in Form von Mikroemulsionen sind ebenfalls neu und Gegenstand der vorliegenden Erfindung.

Die erfindungsgemässen Mikroemulsionen lassen sich aus den erfindungsgemässen Mikroemulsion-Prekonzentraten durch Verdünnen mit Wasser oder anderen wässrigen Flüssigkeiten herstellen. Beim Zusammenbringen des Pre-Konzentrats mit Wasser bzw. Magen- und Darmsaft wird spontan oder praktisch spontan, d.h. ohne nennenswerten Energieeintrag eine Mikroemulsion gebildet.

Je nach der Menge des vorhandenen Wassers handelt es sich um W/O-Mikroemulsionen, um bikontinuierliche Mikroemul-sionen oder um O/W-Mikroemulsionen.

Die erfindungsgemässen Mikroemulsionen vom O/W-Typ (Öl-in-Wasser) weisen Stabilitätseigenschaften auf, wie sie weiter oben im Zusammenhang mit Mikroemulsionen beschrieben worden sind, d.h. insbesondere, dass in diesen Mikroemulsionen der Wirkstoff stabil solubilisiert ist und über mehrere Wochen kein Präzipitat beobachtet werden kann. Die Teilchengrösse dieser Mikroemulsionen ist kleiner als 150 nm, vorzugsweise kleiner als 100 nm. Durch die folgenden Beispiele werden die erfindungsgemässen Zusammensetzungen weiter erläutert. Die Beispiele 1.1 bis 1.3 zeigen die Herstellung von Zusammensetzungen in oralen Einheitsdosierungsformen, die sich beispielsweise zur Prophylaxe oder Therapie von Herz- und Kreislauferkrankungen, degenerativen Erkrankungen des Zentralnervensystems, Zahnfleischerkrankungen, muskulärer Dystrophie, männlicher Infertilität, zur Stärkung des Immun-systems, zur Verbesserung der körperlichen Leistungsfähigkeit und zur Verhinderung oder Reduzierung von Statininduzierten Nebenwirkungen eignen. Beispiel 2.1 zeigt die Herstellung einer Zusammensetzung für eine parenterale Anwendung. In Beispiel 3 wird die orale Bioverfügbarkeit einer erfindungsgemässen Zusammensetzung ermittelt und mit jener von kommerziell erhältlichen Präparaten verglichen.

Die Beispiele werden unter besonderer Bezugnahme auf Coenzym Q10 beschrieben. Durch Anwendung anderer geeigneter Ubiquinone, gegebenenfalls in Kombination mit Vitaminen, vorzugsweise Vitamin E und/oder Spurenelemente lassen sich jedoch vergleichbare Zusammensetzungen herstellen.

### Beispiel 1: Herstellung oraler Coenzym Q10 Dosierungsformen vom Typ Mikroemulsion-Prekonzentrat

### Beispiel 1.1

| | |
|---|---|
| Coenzym Q10 (c1) | 10.00% |
| Miglyol 812 (a1) | 38.90% |
| Oelsäure (a2) | 6.00% |
| Tween 80 (b) | 45.00% |
| Vitamin E (c2) | 0.10% |

Das Coenzym Q10 (c1) wird unter Rühren bei 40 - 45 °C in den Komponenten (a1), (a2), (b) und (c2) gelöst. Das gebildete Mikroemulsion-Prekonzentrat wird in eine Weich- oder Hartgelatinekapsel abgefüllt oder zu Brausetabletten weiterverarbeitet.

Alternativ kann das Mikroemulsion-Prekonzentrat auch in einen Dispenser abgefüllt werden. In diesem Fall stellt der Patient durch entsprechende Verdünnung mittels Wasser oder einer anderen wässrigen Flüssigkeit aus dem Mikroemulsion-Prekonzentrat eine orale Trinklösung vom Typ O/W-Mikroemulsion her.

In analoger Weise lassen sich auch folgende Zusammensetzungen herstellen.

### Referenzbeispiel 1.2

| | |
|---|---|
| Coenzym Q10 (c) | 10.00% |
| Miglyol 812 (a1) | 35.00% |
| Oelsäure (a2) | 10.00% |
| Tween 80 (b1) | 33.75% |
| Cremophor EL (b2) | 11.25% |

### Referenzbeispiel 1.3

| | |
|---|---|
| Coenzym Q10 (c) | 20.00% |
| Miglyol 812 (a1) | 25.00% |
| Oelsäure (a2) | 10.00% |
| Tween 80 (b1) | 33.75% |
| Cremophor EL (b2) | 11.25% |

Werden Zusammensetzungen obiger Art mit Wasser z.B. auf 1:10 verdünnt, entstehen Mikroemulsionen, die folgende Teilchengrössen aufweisen (vgl. Tabelle 1):

**Tabelle 1**

| Zusammensetzung Mikroemulsion-Prekonzentrat | O/W-Mikroemulsion | |
|---|---|---|
| | Partikeldurchmesser¹⁾ [nm] | Standardabweichung¹⁾ [nm] |
| Beispiel 1.1 | 35.7 | 14.2 |
| Beispiel 1.2 | 26.6 | 9.8 |
| Beispiel 1.3 | 28.0 | 10.6 |

| | | |
|---|---|---|
| 1) Die Partikeldurchmesser und Partikelgrössenverteilung wurden mittels dynamischer Laserlicht-Streuungsmessungen bestimmt (Gerät: Nicomp 370 Submicron Particle Sizer, Auswertung: Volume weighting). | | |

Aus der nachfolgenden Tabelle ist ersichtlich, dass die Mikroemulsionsbildung der Mikroemulsion-Prekonzentrate nach Abfüllung sowie Lagerung in Weichgelatinekapseln (WGK) unverändert bleibt.

**Tabelle 2**

| Mikroemulsion-Prekonzentrat Beispiel 1.1 | Partikeldurchmesser der Coenzym Q10 Mikroemulsion¹⁾ | |
|---|---|---|
| | Magensaft [nm] | Darmsaft [nm] |
| Vor Abfüllung in WGK | 41.9 ± 18.1 | 39.0 ± 16.1 |
| Nach Abfüllung in WGK | 41.5 ± 18.9 | 37.8 ± 19.5 |
| Nach 1-monatiger Lagergung in WGK bei 25°C und 60%RH | 45.2 ± 17.9 | 40.6 ± 16.8 |
| Nach 1-monatiger Lagergung in WGK bei 40°C und 75%RH | 44.9 ± 20.2 | 39.5 ± 17.3 |
| Nach 3-monatiger Lagergung in WGK bei 25°C und 60%RH | 43.0 ± 17.6 | 39.4 ± 17.1 |

| | | |
|---|---|---|
| 1) Die Coenzym Q10 Mikroemulsionen wurden durch eine 1:100 Verdünnung der Mikroemulsion-Prekonzentrate mit künstlichem Magen- und Darmsaft bei 37°C gebildet. In WGK abgefüllte Mikroemulsion-Prekonzentrate wurden zur Mikroemulsionsbildung der WGK entnommen. Die Partikel-durchmesser und Partikelgrössenverteilung der erhaltenen Coenzym Q10 Mikroemulsionen wurden mittels dynamischer Laserlicht-Streuungsmessungen bestimmt (Gerät: Nicomp 370 Submicron Particle Sizer, Auswertung: Volume weighting). | | |

### Beispiel 2: Herstellung parenteral anwendbarer Coenzym Q10 Formen vom Typ Mikroemulsion

Die in Beispiel 1.1 bis 1.3 beschriebenen Mikroemulsion-Prekonzentrate können als Basis zur Herstellung von Injektionsoder Infusionslösungen dienen, indem sie mit weiteren Additiven, wie physiologischer Kochsalzlösung oder 5%iger Glukoselösung und dergleichen, entsprechend verdünnt werden.

Referenzbeispiel 2.1: Coenzym Q10 0.10% Infusionslösung
Mikroemulsion-Prekonzentrat gemäss Beispiel 1.2 1.00%
5%ige Glukoselösung ad 100.00%

Das flüssige Mikroemulsion-Prekonzentrat wird unter Rühren bei Raumtemperatur der Glukoselösung zugesetzt. Die resultierende Coenzym Q10 O/W-Mikroemulsion wird 0.2 µm steril filtriert und in gebräuchliche sterile Gebinde abgefüllt.

### Beispiel 3: Bioverfügbarkeit des Coenzym Q10 Mikroemulsion-Prekonzentrates gemäss Beispiel 1.1 nach oraler Verabreichung in Weichgelatinekapsel im Vergleich zu drei kommerziell erhältlichen Präparaten

Ziel dieser vierarmigen, doppelblinden, randomisierter Studie an 20 Probanden beiderlei Geschlechts war es, die Konzentration im Plasma von Coenzym Q10 nach oraler Einmalgabe von 120 mg zu prüfen. Dazu wurde intermittierend über 24 Stunden Blutproben entnommen.

### Präparationen

- A: Weichgelatinekapseln enthaltend das Coenzym Q10
Mikroemulsion-Prekonzentrat gemäss Beispiel 1.1
Charge 201004
Wirkstoffgehalt: 30 mg Coenzym Q10 pro Kapsel
- B: Q-Gel Ultra (Tishcon)
Charge 19710060
Wirkstoffgehalt: 60 mg Coenzym Q10 pro Kapsel
- C: Super Bio-Quinone (Pharma Nord)
Charge 000956
Wirkstoffgehalt: 30 mg Coenzym Q10 pro Kapsel
- D: Bio Coenzyme Q10 (Solanova)
Charge 00310050
Wirkstoffgehalt: 30 mg Coenzym Q10 pro Kapsel

### Dosierung

120 mg Coenzym Q10, oral in 2 bzw. 4 Kapseln

### Einnahme

Die orale Einnahme von 120 mg Coenmzym Q10 erfolgte nüchtern, morgens vor dem Frühstück

### Probanden

n = 20, bei 4 Gruppen zu je 5 Probanden (A - D)

### Messparameter

Plasmaspiegel von Coenzym Q10 [µg/ml Plasma]
Analytik der Plasmaproben

Die quantitative Bestimmung von Coenzym Q10 (Ubidecarenon) erfolgte mittels HPLC

| | |
|---|---|
| Geräte | HPLC-Anlage der Fa. MERCK/HITACHI, UV-Detektion, Autosampler F. Beckmann (Spectra Physics) |
| Trennsäule | Nucleosil RP 18 (5µm), 15 cm Länge, 4 mm Druchmesser, Fa. Merck |
| Elutionsmittel | Acetonitril |
| Dosierschleife | 100/20 µl |
| UV-Detektor | 275 nm |
| Retentionszeit | 10 min |
| Nachweisgrenze | 80 ng/ml |

### Resultate

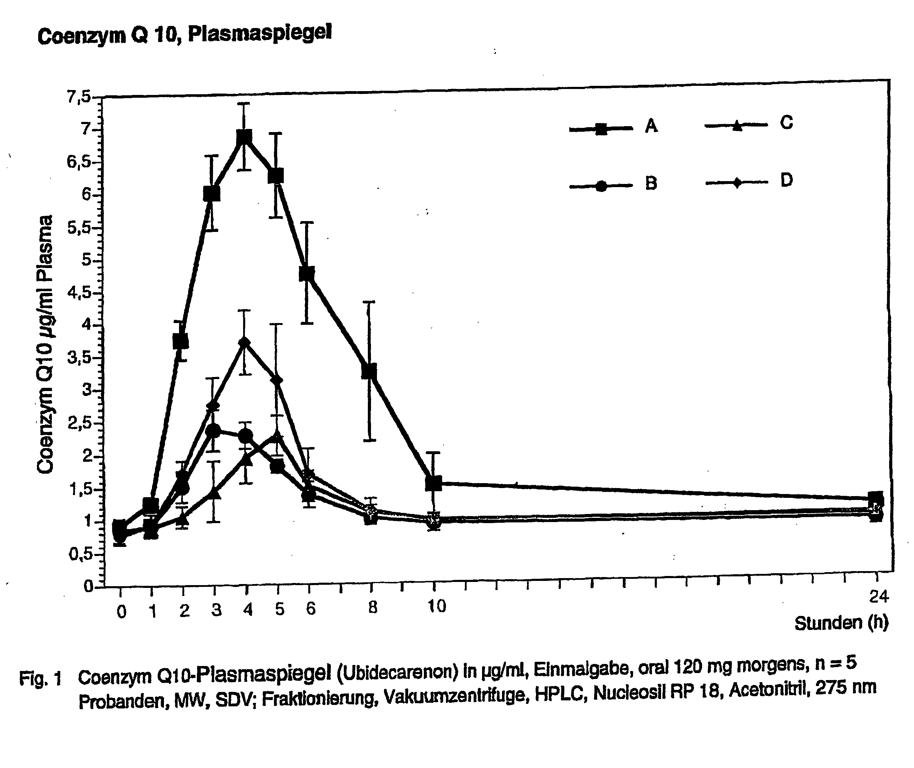

Die Plasmaspiegelverläufe der Präparate A - D zeigen deutliche Unterschiede hinsichtlich des erreichten Maximums und der Anflutungsgeschwindigkeit (vgl. Figur 1). Aus der Berechnung der AUC und der daraus abgeleiteten relativen verfügbaren Dosis, bezogen auf 120 mg Einmalgabe, lassen sich die signifikanten Unterschiede in der Bioverfügbarkeit von Coenzym Q10 nach oraler Einmalgabe deutlich belegen. Die erfindungsgemässe Zusammensetzung (Testpräparat A) weist im Vergleich zu den Testpräparaten B, C und D eine 3-5 fache höhere Bioverfügbarkeit auf (vg. Tabelle 3).

**Tabelle 3**

| Testpräparat | A | B | C | D |
|---|---|---|---|---|
| AUC [µg/ml/10h] | 30.16 | 5.72 | 5.14 | 10.65 |
| Relative verfügbare Dosis bezogen auf 120 mg Einmalgabe | 75.39 | 14.30 | 12.86 | 26.63 |

## Patentansprüche

1. Zusammensetzung in Form eines Mikroemulsion-Prekonzentrates enthaltend
(a) eine Mischung bestehend aus einem Triglycerid, und einer Omega-9-Fettsäure und/oder einer Omega-6-Fettsäure; und
(b) eine oberflächenaktive Komponente enthaltend ein Tensid, insbesondere vom Polyoxyethylen-Typ,
(c) einen Wirkstoffgemisch enthaltend ein Ubiquinon, vorzugsweise Q10, in Kombination mit Vitaminen, vorzugsweise Vitamin E und dessen Derivate, und/oder Spurenelementen, wobei das Ubiquinon in (a) und/oder (b) lösbar ist.

2. Zusammensetzung in Form einer Mikroemulsion erhältlich durch Vermischen eines Mikroemulsions-Prekonzentrats gemäss Anspruch 1 mit Wasser oder einem wässrigen Medium.

3. Zusammensetzung nach Anspruch 1 oder 2, welche im wesentlichen frei ist von mit Wasser mischbaren oder in Wasser löslichen Komponenten.

4. Zusammensetzung nach einem der Ansprüche 1 bis 3, **dadurch gekennzeichnet, dass** die Fettsäurereste des Triglycerids 4-18, vorzugsweise 6-18 C-Atome aufweisen.

5. Zusammensetzung nach Anspruch 4, **dadurch gekennzeichnet, dass** das Triglycerid ein Capryl-/Caprinsäure-Triglycerid ist.

6. Zusammensetzung nach einem der Ansprüche 1 bis 5, **dadurch gekennzeichnet, dass** die Omega-9-Fettsäure und/oder die Omega-6-Fettsäure 12-24, insbesondere 16-24, vorzugsweise 18-22 C-Atome aufweist.

7. Zusammensetzung nach einem der Ansprüche 1 bis 6, **dadurch gekennzeichnet, dass** die Omega-9-Fettsäure Oelsäure ist.

8. Zusammensetzung nach Anspruch 6 oder 7, **dadurch gekennzeichnet, dass** die Omega-6-Fettsäure Linolsäure ist.

9. Zusammensetzung nach einem der Ansprüche 1 bis 8, **dadurch gekennzeichnet, dass** sie als Komponente (a) eine Mischung aus einem Capryl-/Caprinsäure-Triglycerid, Ölsäure und/oder Linolsäure enthält.

10. Zusammensetzung nach einem der Ansprüche 1 bis 9, **dadurch gekennzeichnet, dass** das Mengenverhältnis von Omega-9-Fettsäure und/oder Omega-6-Fettsäure zum Triglycerid 1:1 bis 1:200, vorzugsweise 1:2 bis 1:20 ist.

11. Zusammensetzung nach einem der Ansprüche 1 bis 10, **dadurch gekennzeichnet, dass** die oberflächenaktive Komponente (b) ein Polyoxyethylensorbitanfettsäureester, ein polyoxyethylen-glykoliertes natürliches oder hydriertes Pflanzenöl oder Mischungen davon enthält.

12. Zusammensetzung nach einem der Ansprüche 1 und 3 bis 11, **dadurch gekennzeichnet, dass** die Komponente (a) in einer Menge von 20 bis 70 Gewichtsprozent bezogen auf das Gesamtgewicht der Zusammensetzung vorhanden ist.

13. Zusammensetzung nach einem der Ansprüche 1 und 3 bis 12, **dadurch gekennzeichnet, dass** die oberflächenaktive Komponente (b) in einer Menge von 20 bis 80 Gewichtsprozent bezogen auf das Gesamtgewicht der Zusammensetzung vorhanden ist.

14. Zusammensetzung nach einem der Ansprüche 2 bis 13, **dadurch gekennzeichnet, dass** sie eine O/W-Mikroemulsion mit einer durchschnittlichen Teilchengrösse unter 150 nm, vorzugsweise unter 100 nm ist.

15. Formkörper zur oralen Verabreichung enthaltend eine Zusammensetzung nach einem der Ansprüche 1 und 3 bis 13 zur Darreichung des Wirkstoffs.

16. Formkörper nach Anspruch 15, **dadurch gekennzeichnet, dass** er ein Biopolymer, insbesondere Gelatine, enthält.

17. Brausetablette, enthaltend eine Zusammensetzung nach einem der Ansprüche 1 und 3 bis 13 zur Darreichung des Wirkstoffs.

18. Granulat, enthaltend eine Zusammensetzung nach einem der Ansprüche 1 und 3 bis 13 zur Darreichung des Wirkstoffs.

19. Brausetablette, enthaltend eine Zusammensetzung in Form eines Mikroemulsion-Prekonzentrates enthaltend
(a) eine Mischung bestehend aus einem Triglycerid und einer Omega-9-Fettsäure und/oder einer Omega-6-Fettsäure; und
(b) eine oberflächenaktive Komponente enthaltend ein Tensid, insbesondere vom Polyoxyethylen-Typ,
(c) einen Wirkstoff ausgewählt aus der Klasse der Ubiquinone, wobei der Wirkstoff in (a) und/oder (b) lösbar ist.

20. Granulat, enthaltend eine Zusammensetzung in Form eines Mikroemulsion-Prekonzentrates enthaltend
(a) eine Mischung bestehend aus einem Triglycerid und einer Omega-9-Fettsäure und/oder einer Omega-6-Fettsäure; und
(b) eine oberflächenaktive Komponente enthaltend ein Tensid, insbesondere vom Polyoxyethylen-Typ,
(c) einen Wirkstoff ausgewählt aus der Klasse der Ubiquinone, wobei der Wirkstoff in (a) und/oder (b) lösbar ist.

21. Verwendung eines Formkörpers, Granulats oder einer Brausetablette gemäss einem der Ansprüche 15 bis 20, die sich im Magen-Darmtrakt zersetzen, zur Herstellung eines Arzneimittels zur Freigabe einer mit Magen-Darmsaft spontan eine Mikroemulsion bildenden Zusammensetzung.

## Claims

1. A composition in the form of a microemulsion preconcentrate containing
(a) a mixture comprising a triglyceride and an omega-9-fatty acid and/or an omega-6-fatty acid; and
(b) a surface-active component containing a tenside, in particular of polyoxyethylene type, and
(c) an active substance mixture containing a ubiquinone, preferably Q10, in combination with vitamins, preferably vitamin E and derivatives thereof, and/or trace elements, wherein the ubiquinone is soluble in (a) and/or (b).

2. A composition in the form of a microemulsion which can be obtained by mixing a microemulsion preconcentrate according to claim 1 with water or an aqueous medium.

3. A composition according to claim 1 or claim 2 which is substantially free of components which are miscible with water or soluble in water.

4. A composition according to one of claims 1 to 3 **characterised in that** the fatty acid residues of the triglyceride have 4-18, preferably 6-18 C-atoms.

5. A composition according to claim 4 **characterised in that** the triglyceride is a caprylic/capric acid triglyceride.

6. A composition according to one of claims 1 to 5 **characterised in that** the omega-9-fatty acid and/or the omega-6-fatty acid has 12-14, in particular 16-24, preferably 18-22 C-atoms.

7. A composition according to one of claims 1 to 6 **characterised in that** the omega-9-fatty acid is oleic acid.

8. A composition according to claim 6 or claim 7 **characterised in that** the omega-6-fatty acid is linoleic acid.

9. A composition according to one of claims 1 to 8 **characterised in that** as component (a) it contains a mixture of a caprylic/capric acid triglyceride, oleic acid and/or linoleic acid.

10. A composition according to one of claims 1 to 9 **characterised in that** the quantitative ratio of omega-9-fatty acid and/or omega-6-fatty acid to the glyceride is 1:1 to 1:200, preferably 1:2 to 1:20.

11. A composition according to one of claims 1 to 10 **characterised in that** the surface-active component (b) is a polyoxyethylene sorbitan fatty acid ester, a polyoxyethylene-glycolated natural or hydrated vegetable oil or mixtures thereof.

12. A composition according to one of claims 1 and 3 to 11 **characterised in that** the component (a) is present in an amount of 20 to 70 percent by weight with respect to the total weight of the composition.

13. A composition according to one of claims 1 and 3 to 12 **characterised in that** the surface-active component (b) is present in an amount of 20 to 80 percent by weight with respect to the total weight of the composition.

14. A composition according to one of claims 2 to 13 **characterised in that** it is an O/W-microemulsion with an average particle size of less than 150 nm, preferably less than 100 nm.

15. A shaped body for oral administration containing a composition according to one of claims 1 and 3 to 13 for administering the active substance.

16. , A shaped body according to claim 15 **characterised in that** it contains a biopolymer, in particular gelatin.

17. An effervescent tablet containing a composition according to one of claims 1 and 3 to 13 for administering the active substance.

18. A granular material containing a composition according to one of claims 1 and 3 to 13 for administering the active substance.

19. An effervescent tablet containing a composition in the form of a microemulsion preconcentrate containing
(a) a mixture comprising a triglyceride and an omega-9-fatty acid and/or an omega-6-fatty acid; and
(b) a surface-active component containing a tenside, in particular of polyoxyethylene type, and
(c) an active substance selected from the class of ubiquinones, wherein the active substance is soluble in (a) and/or (b).

20. A granular material containing a composition in the form of a microemulsion preconcentrate containing
(a) a mixture comprising a triglyceride and an omega-9-fatty acid and/or an omega-6-fatty acid; and
(b) a surface-active component containing a tenside, in particular of polyoxyethylene type, and
(c) an active substance selected from the class of ubiquinones, wherein the active substance is soluble in (a) and/or (b).

21. Use of a shaped body, granular material or effervescent tablet according to one of claims 15 to 20 which break down in the gastrointestinal tract, for producing a medicament for release of a composition spontaneously forming a microemulsion with gastrointestinal juice.

## Revendications

1. Composition sous forme d'un préconcentrat d'une microémulsion, contenant
(a) un mélange constitué d'un triglycéride et d'un acide gras oméga-9 et/ou d'un acide gras oméga-6 ; et
(b) un composant à activité de surface, contenant un agent tensioactif, en particulier du type polyoxyéthylène,
(c) un mélange à base d'une substance active, contenant une ubiquinone, de préférence la Q10, en combinaison avec des vitamines, de préférence la vitamine E et ses dérivés, et/ou des éléments à l'état de traces, l'ubiquinone étant soluble dans (a) et/ou (b).

2. Composition sous forme d'une microémulsion pouvant être obtenue par mélange d'un préconcentrat d'une microémulsion selon la revendication 1 avec de l'eau ou un milieu aqueux.

3. Composition selon la revendication 1 ou 2, qui est essentiellement exempte de composants miscibles à l'eau ou solubles dans l'eau.

4. Composition selon l'une quelconque des revendications 1 à 3, **caractérisée en ce que** les restes acide(s) gras du triglycéride présentent 4 à 18, de préférence 6 à 18, atomes de carbone.

5. Composition selon la revendication 4, **caractérisée en ce que** le triglycéride est un triglycéride d'acide caprique et d'acide caprylique.

6. Composition selon l'une quelconque des revendications 1 à 5, **caractérisée en ce que** l'acide gras oméga-9 et/ou l'acide oméga-6 présentent 12 à 24, en particulier 16 à 24, de préférence 18 à 22, atomes de carbone.

7. Composition selon l'une quelconque des revendications 1 à 6, **caractérisée en ce que** l'acide gras oméga-9 est l'acide oléique.

8. Composition selon la revendication 6 ou 7, **caractérisée en ce que** l'acide gras oméga-6 est l'acide linoléique.

9. Composition selon l'une quelconque des revendications 1 à 8, **caractérisée en ce qu'**elle contient à titre de composant (a) un mélange d'un triglycéride de l'acide caprique et de l'acide caprylique, d'acide oléique et/ou d'acide linoléique.

10. Composition selon l'une quelconque des revendications 1 à 9, **caractérisée en ce que** le rapport de mélange des acides gras oméga-9 et/ou oméga-6 au triglycéride est de 1:1 à 1:200, de préférence de 1:2 à 1:20.

11. Composition selon l'une quelconque des revendications 1 à 10, **caractérisée en ce que** le composant à activité de surface (b) est un ester d'acide gras et de polyoxyéthylène sorbitane, une huile végétale naturelle ou hydrogénée polyoxyéthylène-glycolysée ou des mélanges de ceux-ci.

12. Composition selon l'une quelconque des revendications 1 et 3 à 11, **caractérisée en ce que** le composant (a) est présent en une quantité de 20 à 70% en poids par rapport au poids total de la composition.

13. Composition selon l'une quelconque des revendications 1 et 3 à 12, **caractérisée en ce que** le composant à activité de surface (b) est présent en une quantité de 20 à 80% en poids par rapport au poids total de la composition.

14. Composition selon l'une quelconque des revendications 2 à 13, **caractérisée en ce qu'**il s'agit d'une microémulsion H/E présentant une grosseur moyenne de particule inférieure à 150 nm, de préférence inférieure à 100 nm.

15. Corps façonné pour l'administration orale contenant une composition selon l'une quelconque des revendications 1 et 3 à 13 pour l'administration de la substance active.

16. Corps façonné selon la revendication 15, **caractérisé en ce qu'**il contient un biopolymère, en particulier la gélatine.

17. Comprimé effervescent contenant une composition selon l'une quelconque des revendications 1 et 3 à 13 pour l'administration de la substance active.

18. Granulat contenant une composition selon l'une quelconque des revendications 1 et 3 à 13 pour l'administration de la substance active.

19. Comprimé effervescent contenant une composition sous forme d'un préconcentrat d'une microémulsion contenant
(a) un mélange constitué d'un triglycéride et d'un acide gras oméga-9 et/ou d'un acide gras oméga-6 ; et
(b) un composant à activité de surface contenant un agent tensioactif, en particulier du type polyoxyéthylène,
(c) une substance active choisie dans la classe des ubiquinones, la substance active étant soluble dans (a) et/ou (b).

20. Granulat contenant une composition sous forme d'un préconcentrat d'une microémulsion contenant
(a) un mélange constitué d'un triglycéride et d'un acide gras oméga-9 et/ou d'un acide gras oméga-6 ; et
(b) un composant à activité de surface contenant un agent tensioactif, en particulier du type polyoxyéthylène,
(c) une substance active choisie dans la classe des ubiquinones, la substance active étant soluble dans (a) et/ou (b).

21. Utilisation d'un corps façonné, d'un granulat ou d'un comprimé effervescent selon l'une quelconque des revendications 15 à 20, qui se décompose dans le tractus gastrointestinal, pour la préparation d'un médicament destiné à la libération d'une composition formant spontanément une microémulsion avec le suc gastrointestinal.
